# EUROPEAN PATENT APPLICATION

(11) **EP 4 826 736 A1**
(43) Date of publication of application: **21.10.2026**
(21) Application number: 25171409.3
(22) Date of filing: 17.04.2025
(51) Int. Cl.: G16H 50/20, G16H 20/40

(54) **FISTULA FAILURE PREDICTION**

(71) Applicant: Fresenius Medical Care Deutschland GmbH, 61352 Bad Homburg (DE)
(72) Inventor: Neri, Luca, 61352 Bad Homburg (DE); Bregoli, Alessandro, 61352 Bad Homburg (DE); Maierhofer, Andreas, 61352 Bad Homburg (DE); Kopperschmidt, Pascal, 61352 Bad Homburg (DE); Bellocchio, Francesco, 61352 Bad Homburg (DE)
(74) Representative: Bobbert & Partner Patentanwälte PartmbB

(57) **Abstract**

The present invention relates to a computer-implemented method for predicting a patient's (P) fistula failure encompassing an inputting of a plurality of parameter values, into a computer system (1), wherein some of these values have been measured during one or more dialysis treatment sessions while treating the patient (P) using a hemodialysis treatment apparatus (100) and/or wherein some of these values are or have been calculated based on or derived from values having been measured during one or more dialysis treatment sessions while treating the patient (P) using a hemodialysis treatment apparatus (100). The computer-implemented method further encompasses a predicting of the probability of an event predefined as a fistula failure within a predetermined time period or within a predetermined number of treatment sessions. Moreover, it encompasses an outputting of the predicted probability.

## Description

The present invention relates to a computer-implemented method for predicting a patient's fistula or vascular access failure, in particular venous access failure according to claim 1, a computer system according to claim 8, and a hemodialysis treatment apparatus according to claim 10. Furthermore, it relates to a digital storage medium according to claim 15, a computer program product according to claim 16, and a computer program according to claim 17 or according to the preambles or generic terms of each of these claims.

For patients with chronic kidney disease (CKD) the renal replacement therapy (RRT) is an essential treatment. Hemodialysis is one of the most commonly used forms of RRT and requires well-functioning vascular access for administration. Accordingly, an arteriovenous fistula (AVF) is one of the most commonly used types of vascular accesses, especially in Europe. As a result, significant efforts have been made to ensure the survival and patency of AVFs. Medical literature suggests that the physical examination is an effective and inexpensive method for monitoring the AVFs. This is, however, time-consuming. The same applies for so-called arteriovenous (AV) grafts, short: AV-grafts, which resemble the arteriovenous fistula in many ways but encompass graft material (made from PTFE or the like). Since such grafts may encounter identical or similar problems as the arteriovenous fistula does, such grafts are considered hereinafter as well. Also, some patients are treated - mostly on acute care wards - using a central venous catheter or line. Those accesses to the patient's venous system are also considered hereinafter. Hence, if an arteriovenous fistula is addressed herein, the respective disclosure and protection by the claims attached shall not be limited to arteriovenous fistula as such but in some embodiments extended to arteriovenous (AV) grafts, central venous catheters and/or any other type of vascular access or catheter or to the prediction of failure of the same.

An object of the present invention is to suggest a computer-implemented method for predicting a patient's fistula failure, a further computer system as well as a further hemodialysis treatment apparatus. Moreover, a digital storage medium, a computer program product, and a computer program should be suggested.

The objective of the present invention may be achieved by the computer-implemented method for predicting a patient's fistula failure having the features of claim 1, the computer system having the features of claim 8, and the hemodialysis treatment apparatus having the features of claim 10. It may further be achieved by the digital storage medium having the features of claim 15, by the computer program product having the features of claim 16, as well as by the computer program having the features of claim 17.

According to the present invention, a computer-implemented method for predicting a patient's fistula failure or for assessing the probability of the fistula to fail is suggested. Thereby, the probability to fail may be considered, e.g., with regard to a preset time period or to the course of pre-determined number of treatment sessions, or the like.

The computer-implemented method comprises an inputting of a plurality of parameter values into a computer system or a component thereof. Some of these values may have been measured during one or more dialysis treatment sessions while treating the patient using a hemodialysis treatment apparatus or in connection with such dialysis treatment sessions, respectively. Additionally or alternatively, some of these values may be or may have been calculated based on or derived from values having been measured during one or more dialysis treatment sessions while treating the patient using the hemodialysis treatment apparatus.

Furthermore, the computer-implemented method comprises predicting or assessing the probability or estimated probability of an event predefined as a fistula failure within a predetermined time period. This time period may start as of the time of the prediction or as of the time of executing/carrying out, in particular starting, of this method. This time period may cover a predetermined number of, preferably subsequent, treatment sessions.

Eventually, the computer-implemented method comprises that the predicted probability or the result of the assessment is output.

The predicted probability may in some embodiments be considered the target variable or the dependent variable in statistics. A target variable is the outcome one aims to predict or explain using, e.g., a machine learning model. A target variable is the variable that is estimated or classified based on the input values. The result of the assessment may be a simple "true" or "false", e.g., with respect to a probability hypothesis.

In some embodiments, outputting the predicted probability or the result of the assessment may refer to an information leaving the computer system on which the method according to the invention is carried out. It may, for example be displayed, sent, printed, or the like such that a person or an apparatus not pertaining to the computer system is provided with the predicted probability or the result. In certain embodiments, however, outputting means that the knowledge of the probability or the result does not leave the system or may not be received by a person or an apparatus foreign to the system, or not exclusively. In some embodiments, outputting the predicted probability or the result of the assessment may refer to both providing the information to an outside world to the computer system and effecting changes in the control of an hemodialysis treatment apparatus, e.g. via its component needed for treating the patient.

According to the present invention, a computer system is suggested that is programmed for carrying out/executing the computer-implemented method according to the present invention.

Furthermore, according to the present invention, a hemodialysis treatment apparatus is suggested. The hemodialysis treatment apparatus comprises components for carrying out a hemodialysis treatment and a control unit for controlling the components.

The apparatus further comprises the computer system according to the present invention or is in signal communication with it. Alternatively, it is prepared for being in signal communication with it.

A digital, particularly non-volatile storage medium, according to the present invention, particularly in the form of a machine readable carrier, particularly in the form of a diskette, floppy disk, memory card, CD, DVD EPROM, FRAM (Ferroelectric RAM) or SSD (Solid-State-Drive), particularly with electronically or optically readable control signals may interact with a conventional computer system such that it is reprogrammed into a computer system according to the present invention and/or interact with the controller of a conventional hemodialysis treatment apparatus such that it is turned, converted, changed, transformed or reprogrammed into a hemodialysis treatment apparatus according to the present invention.

A computer program product according to the present invention comprises a volatile or transient program code or one stored on a machine-readable carrier or a signal wave, with which a conventional computer system is reprogrammed into a computer system according to the present invention and/or the controller of a conventional hemodialysis treatment apparatus is reprogrammed into a hemodialysis treatment apparatus according to the present invention when the computer program product runs on a computer.

A computer program product can be, for example, understood according to the present invention as a computer program stored on a carrier, an embedded system being a comprehensive system with a computer program (e.g., an electronic device with a computer program), a network of computer implemented computer programs (e.g. client/server-system, a cloud computing system etc.), or a computer on which a computer program is loaded, runs, is stored, is being executed or developed.

The term "machine readable carrier" as used herein, refers in certain embodiments of the present invention to a carrier, which contains data or information interpretable by software and/or hardware. The carrier may be a data carrier, such as a diskette, a CD, DVD, a USB stick, a flashcard, an SD card or the like, as well as any other storage referred to herein or any other storage medium referred to herein.

A computer program according to the present invention comprises a program code with which, when executed on a computer, a conventional computer system is reprogrammed into a computer system according to the present invention and/or with which the controller of a conventional hemodialysis treatment apparatus such that the conventional hemodialysis treatment apparatus is turned, converted, changed, transformed or reprogrammed into a hemodialysis treatment apparatus according to the present invention.

The herein discussed computer-implemented method for predicting a patient's fistula failure is subject-matter of the present invention and, therefore, according to the present invention. The herein discussed computer system is subject-matter of the present invention and, therefore, according to the present invention. The herein discussed hemodialysis treatment apparatus is subject-matter of the present invention and, therefore, according to the present invention. The herein discussed digital storage medium is subject-matter of the present invention and, therefore, according to the present invention. The herein discussed computer program product is subject-matter of the present invention and, therefore, according to the present invention. The herein discussed computer program is subject-matter of the present invention and, therefore, according to the present invention.

Embodiments according to the present invention may comprise and/or consist of one or several of the features mentioned supra and/or in the following, e.g. based on any of the independent claims. Thereby, the herein mentioned features may be the subject-matter in any combination based on any of the independent claims or any part or combination of features or sub-combinations thereof, unless the person skilled in the art considers the particular combination to be technically impossible.

Advantageous developments of the present invention are each subject-matter of the dependent claims and embodiments.

In all of the aforementioned and following statements, the use of the expression "may be" or "may have" and so on, is to be understood synonymously with "preferably is" or "preferably has", and so on respectively, and is intended to illustrate an embodiment according to the present invention.

Whenever alternatives with "and/or" are introduced herein, the person skilled in the art will understand the "or" contained therein as preferably "either or" and preferably not as "and".

Whenever numerical words are mentioned herein, the person skilled in the art shall recognize or understand them as indications of numerical lower limits. Hence, unless this leads to a contradiction evident for the person skilled in the art, the person skilled in the art shall comprehend for example "one" (or "a/an") as encompassing "at least one". This understanding is also equally encompassed by the present invention as the interpretation that a numerical word, for example, "one" (or "a/an") may alternatively mean "exactly one", wherever this is evidently technically possible in the view of the person skilled in the art. Both of these understandings are encompassed by the present invention and apply herein to all used numerical words.

Whenever an embodiment is mentioned herein, it represents an exemplary embodiment according to the present invention, for example, based on one of the independent claims, and is not to be understood as limiting.

When it is disclosed herein that the subject-matter according to the present invention comprises one or several features in a certain embodiment, it is also respectively disclosed herein that the subject-matter according to the present invention does, in other embodiments, likewise according to the present invention, explicitly not comprise this or these features, for example, in the sense of a disclaimer. Therefore, for every embodiment mentioned herein it applies that the converse embodiment, e.g. formulated as negation, is also disclosed.

When method steps are mentioned herein, then the devices according to the present invention are in several embodiments configured in order to execute, in any combination, one or several of these method steps, in particular when these are automatically executable steps, or in order to accordingly control corresponding apparatuses which preferably correspond with their names to the designation of the respective method step (for example, "determining" as a method step and "apparatus for determining" for the apparatus) and which may likewise be part of the apparatus(es) according to the present invention or connected thereto in signal communication.

When programmed or configured is mentioned herein, then these terms may in some embodiments be interchangeable.

When a signal communication or communication connection between two components is mentioned herein, this may be understood to mean a connection that exists during use. It may also be understood that a preparation for such a (wired, wireless or otherwise implemented) signal communication exists, for example by coupling both components, for example by pairing, etc.

Pairing is a process that takes place in connection with computer networks in order to establish an initial link between computer units for the purpose of communication. The best-known example of this is the establishing of a Bluetooth connection, by which various devices (e.g. smartphone, headphones) are connected to each other. Pairing is sometimes also referred to as bonding.

When method steps are mentioned herein, then it is provided in several embodiments according to the present invention that these steps are carried out prior to or after a treatment of the patient, for instance during the time when the patient is not connected, e.g. via the extracorporeal blood circuit or the like, to the apparatus or that a treatment has not begun yet or is already completed.

The treatment of the patient may at least be completed or may not have started yet, when, e.g., the patient is not connected to the hemodialysis treatment apparatus, e.g., because their vascular system is not connected to an extracorporeal blood circuit in fluid communication, or when no blood or no liquid is returned into their vascular system, in particular not at the relevant point in time and/or not anymore in the further course of the relevant treatment session.

The computer system may be, or comprise, a control device or closed-loop control device that may prompt the execution of all or substantially all of the method steps. The method according to the present invention may substantially or completely be executed by the control device or closed-loop control device. It may be partly executed by the control device or closed-loop control device. Such method steps may optionally be restricted to machine-induced or automatic method steps.

In some embodiments the verbs "turn", "convert", "change", "transform" or "reprogram" may be used interchangeably.

In several embodiments of the computer-implemented method according to the present invention, the values of the parameters which are input comprise or consist of, at least two or more of, the following ones:
- clearance
- sum pressure
- clearance/blood flow-ratio
- clearance/reference clearance-ratio

The clearance is the capacity of the dialyzer to clean the blood of *urea,* or is the urea removal rate, and can be calculated based on urea measurements, preferably in as a in-vivo clearance measurement. For example, the Online Clearance Monitoring (OCM^{®}) system by Fresenius Medical Care, Germany, integrated, e.g., into their therapy system 5008, intra-dialytically measures the effective in-vivo urea clearance achieved during the treatment session.

To determine the clearance, a control and evaluation unit, means to influence the dialysate composition to induce a change of the conductivity at the dialyzer inlet, to evaluate the "response" at the dialyzer outlet, and to gather a value for the electrolyte clearance, which is representative of the urea clearance, may be used. Of course, there are other methods known for measuring clearance, such as spectroscopy, which do not require inducing changes to the dialysate properties or composition.

Generally spoken, for determining the clearance a property of the used dialysate fluid is preferably measured. However, the property of interest does not necessarily have to be urea. Rather, the findings related the clearance of the property or urea may be used for determining the clearance in general or of interest.

EP 0 845 273 A1 discloses a method for determining the clearance (also referred to as dialysance D therein) for a mathematical determination of the clearance of the method or the system, in particular the hemofilter, used when treating the patient via dialysis. The same holds true for WO 2023/062047 A1. The respective disclosures of EP 0 845 273 A1 and WO 2023/062047 A1 are incorporated herein by way of reference, both regarding the methods and the devices disclosed there.

In some embodiments, the reference clearance is an expected clearance based on treatment parameters like inter alia mass transfer-area coefficient (k0A) of the used dialyzer, blood flow rate of the extracorporeal blood circuit, dialysis fluid flow rate and treatment mode (hemodialysis, hemodiafiltration with post or pre dilution etc.), as disclosed in WO 2023/062047 A1, while the clearance is the measured clearance of the actual treatment as determined e.g. as disclosed in EP 0 845 273 A1.

In certain embodiments, the following formula, found and explained in more detail in WO 2023/062047 A1, is used for calculating the reference clearance K_{ref}: $\begin{matrix}{K}_{ref} = \frac{{Q}_{b}}{{W}_{b} + {κQ}_{s}} \left({D}_{diff}\frac{{Q}_{b} - {Q}_{f} - \left(1-κ\right) {Q}_{s}}{{Q}_{b} + {κQ}_{s}}+{Q}_{f}+{Q}_{s}\right) \\ κ = \left\{\begin{matrix}0 & HD , HDF - post \\ 1 & HDF - pre\end{matrix}\right.\end{matrix}$ with
- Q_{b}: being the blood flow rate
- Qₛ: being the substitute fluid flow rate
- Q_{f}: being the ultrafiltration rate
- D_{diff}: being the diffusive part of Clearance K
- HD: referring to hemodialysis
- HDF - post: referring to hemodiafiltration with post-dilution
- HDF - pre: referring to hemodiafiltration with pre-dilution

The concept of measuring the clearance is well described in DRUKKER, PARSONS and MAHER, "Replacement of Renal Function by Dialysis", 5th edition, Kluwer Academic Publishers, Dordrecht/Boston/London 2004, ISBN 1-4020-0083-9, see in particular page 280, 414f and chapter 22. The respective disclosure thereof is incorporated herein by way of reference.

The sum pressure is the sum of the, e.g. simultaneously, measured arterial pressure and venous pressure, e.g. the pressure measured by sensors already present within the hemodialysis treatment apparatus.

The clearance/blood flow-ratio is the clearance divided by the blood flow or vice versa, and the clearance/reference clearance-ratio is the clearance divided by a reference clearance, or vice versa. The reference clearance may be pre-set or stored or looked-up from, e.g. a table.

There are several methods for determining the reference clearance: based on a model of the dialysis system (including, e. g., the dialyzer and the patient with recirculation), based on previous measurements, estimates from comparable cohorts, and others.

In general, the reference clearance, as referred to herein, may be derived at using the same methods or devices as used for deriving at the clearance as referred to herein.

Patent application WO 2023/062047 A1 discloses a number of methods for determining or estimating a clearance or a reference clearance, and further refers to additional methods not or not fully described in said patent application, all of which can be used for determining or estimating a clearance and/or a reference clearance for the purpose of this invention. The respective disclosure of WO 2023/062047 A1 and the sources referred to by it are incorporated herein by way of reference.

In particular, reference values can be patient-specific, derived from current or past treatments. Alternatively, global, center-specific, or organization-specific values can also be used. These values may be available locally or distributed (e.g., in the cloud) and can be made available for analysis before or during treatment.

In some embodiments, the dynamic of the parameters during a treatment session of interest is not taken into account, but, for example, the median value of said parameter values measured during or calculated with respect to the treatment session.

In several embodiments, the predicted probability is output to a printer, a display, a handheld device of any kind, e.g. a smartphone, a tablet etc., and/or to the hemodialysis treatment apparatus.

In some embodiments, an artificial intelligence model, e.g., a machine learning model, is used for predicting the probability.

In several embodiments, a logistic regression model is used. This model estimates the probability of failure based on a linear combination of the input variables. Alternatively, a gradient boosting framework based on decision tree classifiers is used for predicting the probability, e.g. the LightGBM algorithm as shown in Guolin Ke et al. "LightGBM: A Highly Efficient Gradient Boosting Decision Tree" in: Advances in Neural Information Processing Systems, Ed. by I. Guyon et al, Vol. 30, Curran Associates, Inc., 2017. This is a more sophisticated model, if compared with the logistic regression, capable to deal with non-linear relation between the input variables and the target.

Experiments by the inventors have shown that the two above-mentioned approaches identify risk class with similar risk of fail. The main difference is observed in the high risk class (the 0.1% hemodialysis treatment sessions with the highest risk of failure), where the logistic regression is more effective in identify fistulas that will fail within a month.

In several embodiments, the computer-implemented method encompasses outputting suggestions regarding medical or surgical treatment based on the predicted probability or the result.

In some embodiments of the hemodialysis treatment apparatus, the control unit comprises or consists of the computer system, or vice versa.

In several embodiments, the hemodialysis treatment apparatus further comprises measuring devices for measuring the parameters the values of which are input. Additionally or alternatively, the hemodialysis treatment apparatus comprises a calculating device for calculating a value of at least one of the parameters.

In some embodiments, the hemodialysis treatment apparatus is a self-contained system. It may comprise all means necessary to carry out the computer-implemented method according to the invention. In particular, it may comprise all means necessary to measure the parameter values at issue or the values the parameter of interest are based on.

In some embodiments, the control unit is programmed to control the hemodialysis treatment apparatus during treatment based on the predicted probability output.

For example, in some embodiments, the control unit is programmed to limit the allowable blood flow rate that the doctor may set for treatment, both during an ongoing treatment session and for a treatment session yet to be started.

Also, in certain embodiments, the control unit is programmed to adjust automatically one or more of the set treatment parameters based on the predicted probability or the result. For example, the predicted probability or the result may be compared to pre-set thresholds, reference probabilities or results, classes of reference probabilities or results or the like which may be stored at a place accessible to the computer system, e.g. look-up table, a storage, or the like. Depending on this comparison, a pump rate may automatically be lowered, a treatment time prolonged, and so on. The extent to which this happens may be stored and, e.g., be linked to specific probabilities or results.

In several embodiments, the predicted probability or the result of the assessment may trigger outputting measures or advices as to how to deal with the fistula state assumed based on the probability or result, e.g. via a printer, a monitor, to a computer system, a hand-held apparatus like a smart phone, and the like.

The measures or advice may comprise a suggestion such as a surgical intervention, a check-up at the specialist, a stenting procedure of the fistula, and so on. A look-up table or a storage may be provided that allots particular measures to particular probabilities or results.

In some embodiments, the control unit is programmed to output suggestions regarding medical or surgical treatment based on the predicted probability or the result. This may be another step of the method according to the invention as well.

Optionally, the method steps described herein may be carried out in the above-mentioned order, with a time overlap or simultaneously.

In some embodiments, the hemodialysis treatment apparatus or any other device disclosed herein comprises, for determining the clearance or the reference clearance, all means necessary. Those means may encompass a control and evaluation unit (which may be the control unit disclosed herein), a device for influencing the dialysate composition at the dialyzer inlet to induce a change of the conductivity of the dialysate, a device for evaluating of the "response" that can be measured in the dialysate at the dialyzer outlet, and a device to gather a value for the clearance, which may be a value of urea or any other suitable substance, in particular electrolyte.

In particular embodiments, the hemodialysis treatment apparatus or any other device disclosed herein is configured for carrying out any method disclosed or mentioned herein for determining the clearance of urea or any other suitable substance, in particular electrolyte.

It may, for example, comprise a device for measuring clearance by means of spectroscopy.

In certain embodiments, the hemodialysis treatment apparatus or any other device disclosed herein is configured for carrying out any method disclosed or mentioned herein for determining - or retrieving (form a memory, e.g.) the reference clearance as mentioned herein.

Some or all of the embodiments according to the present invention may comprise one, several or all of the advantages mentioned above and/or in the following.

As the present invention is capable of predicting the risk of failure of a vascular access, this may advantageously be extremely valuable for clinician and patients. Patient safety may be substantially increased.

As in some (or all) embodiments the computer-implemented method according to the present invention uses only data already collected by the hemodialysis treatment apparatus to develop an ML model for predicting the risk of failure of the fistula, it is relatively easy to implement the method with common hemodialysis treatment machines. This may help to save manpower and money.

Even with only a limited number of parameters considered and using a simple model such as the logistic regression, it is possible to achieve reasonable performance. Therefore, it is possible to implement an ML model directly into the control unit or any other part of, or related to, the hemodialysis treatment apparatus, which allows for timely alert to be delivered and, therefore, to increase patient safety.

All the advantages achievable with the method according to the present invention can be in certain embodiments achieved undiminished by the apparatus and/or devices according to the present invention and vice versa.

In the following, the present invention is described based on preferred embodiments thereof with reference to accompanying drawing. However, the present invention is not to be limited to these embodiments. In the figures, in which same reference numerals denote identical or similar elements, the following applies:
- **Fig. 1**: shows, schematically simplified, a fluid line diagram of a hemodialysis treatment apparatus according to the present invention in an exemplary embodiment; and
- **Fig. 2**: shows a flowchart of a computer-implemented method according to the present invention in an exemplary embodiment.

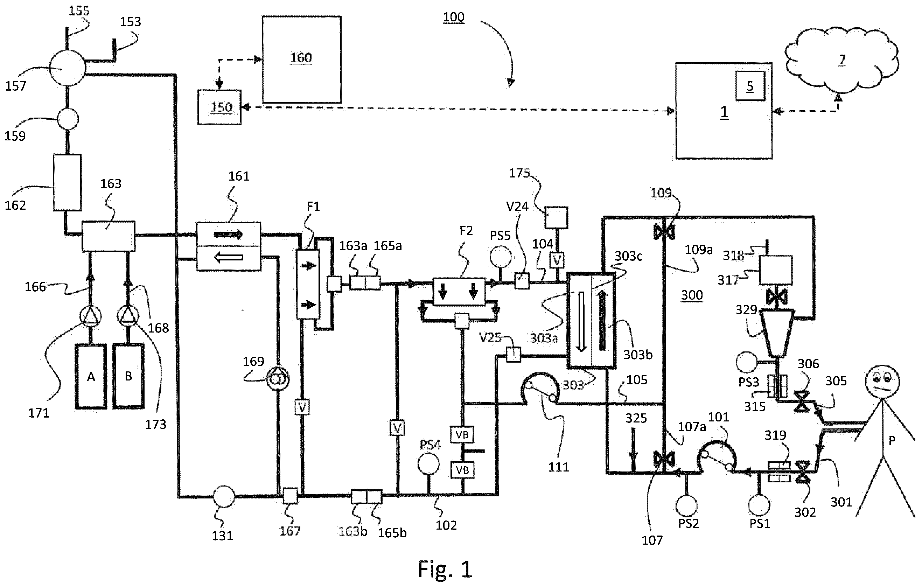

**Fig. 1** shows, schematically simplified, a fluid line diagram of a hemodialysis treatment apparatus 100 according to the present invention in an exemplary embodiment.

The hemodialysis treatment apparatus 100, shown in an at least partially equipped state of use, is connected to an extracorporeal blood circuit 300, which may be connected for treatment to the vascular system of the patient P using double-needle access as shown in Fig. 1, or by using e.g. an additional Y-connector , for single-needle access or by using a central venous catheter or any other vascular access (or device, respectively), wherein said extracorporeal blood circuit 300 is optionally not part of the hemodialysis treatment apparatus 100, whereas it is in other embodiments. The blood circuit 300 may be present, optionally in sections thereof, in or on a blood cassette.

Pumps, actuators and/or valves in the area of the blood circuit 300 are in signal communication with the treatment apparatus 100 according to the present invention or with a control device 150, e.g. comprised by it, where required for controlling it or controlling it in a closed-loop manner.

The blood circuit 300 comprises (or is connected to) an arterial patient tubing clamp 302 and an arterial connection needle of an arterial section or of an arterial patient line, blood withdrawal line or first line 301. The blood circuit 300 also comprises (or is connected to) a venous patient tubing clamp 306 and a venous connection needle of a venous section, a venous patient line, blood return line or second line 305.

A blood pump 101 is provided in or at the first line 301, an optional substitute fluid pump 111 is connected to, if applicable, a dialysis liquid inlet line 104 for conveying fresh dialysis liquid, which may be filtered in an optional further filter stage F2 (substitute fluid). A substitute fluid line 105 may be fluidically connected to the dialysis liquid inlet line 104. Using the optional substitute fluid pump 111, substitute fluid may be introduced by pre-dilution, via a pre-dilution valve 107, or by post-dilution, via a post-dilution valve 109, via associated lines 107a or 109a into line sections, for example, into the arterial line section 301 or into the venous line section 305 (here between a blood chamber 303b of a blood filter 303 and a venous air separation chamber or venous bubble trap 329) of the blood circuit 300.

The blood filter 303 comprises the blood chamber 303b connected to the arterial line section 301 and to the venous line section 305. A dialysis liquid chamber 303a of the blood filter 303 is connected to the dialysis liquid inlet line 104 leading to the dialysis liquid chamber 303a and to a dialysate outlet line 102, leading away from the dialysis liquid chamber 303a, which dialysate outlet line 102 guides dialysate, i.e. spent dialysis liquid. Suitable connectors at the dialysis liquid inlet line 104 or at the dialysate outlet line 102 on the one hand and at the dialysate ports on the other, which may be, in particular releasably, connected to each other serve for this purpose.

Dialysis liquid chamber 303a and blood chamber 303b are separated from each other by a mostly semi-permeable membrane 303c. It represents the partition between the blood side with the extracorporeal blood circuit 300 and the machine side with the dialysis liquid or dialysate circuit, which is shown in Fig. 1 to the left of the membrane 303c.

The arrangement of Fig. 1 further comprises a valve V24 arranged in the dialysis liquid inlet line 104 upstream of the blood filter 303, but downstream of a pressure sensor PS5. It further comprises a valve V25 arranged in the dialysate outlet line 102, downstream of the blood filter 303, but upstream of another pressure sensor PS4.

The arrangement of Fig. 1 comprises an optional detector 315 for detecting air and/or blood. The arrangement in Fig. 1 further comprises one or two optional pressure sensors PS1 (upstream of the blood pump 101) and PS2 (downstream of the blood pump 101), it measures the pressure upstream of the blood filter 303 ("pre-hemofilter") at the points shown in Fig. 1. Further pressure sensors may be provided, e.g. the pressure sensor PS3 downstream of venous bubble trap 329.

Blood exiting the blood filter 303 flows through the optional venous bubble trap 329, which may comprise a de-aeration device 318 and be in fluid communication with the pressure sensor PS3.

An optional single-needle chamber 317 is used in Fig. 1 as a buffer and/or compensating reservoir in a single-needle in which the patient is connected to the extracorporeal blood circuit 300 using only one of the two blood lines 301, 305.

The arrangement of Fig. 1 also comprises an optional detector 319 for detecting air bubbles and/or blood.

An addition site 325 for Heparin or for other anticoagulants may optionally be provided.

On the left in Fig. 1, there is shown an mixing device 163, which provides a predetermined mixture for the respective solution from the containers A (for A-concentrate via concentrate supply 166) and B (for B-concentrate via concentrate supply 168) for use by the hemodialysis treatment apparatus 100. The solution contains water from the water source 155 (on-line, e.g. as reverse osmosis water or from bags) which is heated, for example, in the heating device 162.

A pump 171, which may be referred to as concentrate pump or sodium pump, is in fluid communication with the mixing device 163 and a source of sodium, for instance the container A, and/or conveys out of it. An optional pump 173, associated with container B, for instance for bicarbonate, can also be seen.

Furthermore, Fig. 1 shows an outlet 153 for the effluent. An optional heat exchanger 157 and a first flow pump 159, which is suitable for degassing, complete the arrangement shown.

The pressure sensor PS4 downstream of the blood filter 303 on the water side, but preferably upstream the ultrafiltration pump 131 in the dialysate outlet line 102 may be provided for measuring the filtrate pressure or membrane pressure of the blood filter 303.

The exemplary arrangement shown in Fig. 1 depicts the control device 150. The latter may be in a wired or wireless signal connection with any of the components mentioned herein - especially or in particular with the blood pump 101 - to control or control in a closed-loop manner the hemodialysis treatment apparatus 100.

By using the device for on-line mixing of the dialysis liquid, a variation of its sodium content, controlled by the control device 150, is possible within certain limits. For this purpose, in particular the measurement values determined by the conductivity sensors 163a, 163b may be taken into account. Should an adjustment of the sodium content of the dialysis liquid (sodium concentration) or of the substitute fluid turn out to be necessary or desired, this can be done by adjusting the conveying rate of the sodium pump 171.

In addition, the treatment apparatus 100 comprises devices for conveying fresh dialysis liquid and dialysate. A first valve, such as valve V24, may be provided between the first flow pump 159 and the blood filter 303, which first valve opens or closes the inflow towards the blood filter 303 at the inlet side. A second, optional flow pump 169 which conveys dialysate towards the outlet 153 is provided, e.g., downstream of the blood filter 303. A second valve, such as valve V25, may be provided between the blood filter 303 and the second flow pump 169, which second valve opens or closes the outflow at the outlet side.

Furthermore, the treatment apparatus 100 optionally comprises a device 161 for balancing the flow flowing into and out of the dialyzer 303 on the machine side. The device 161 for balancing is preferably arranged in a line section between the first flow pump 159 and the second flow pump 169.

The treatment apparatus 100 further comprises means, such as the ultrafiltration pump 131, for the precise removal of a volume of liquid from the balanced circuit, as preset by the user and/or by the control device 150.

Sensors such as the optional conductivity sensors 163a, 163b serve to determine the conductivity, which in some embodiments is temperature-compensated, as well as the liquid flow upstream and downstream of the dialyzer 303.

Temperature sensors 165a, 165b may be provided individually or in groups. Temperature values supplied by them may be used to determine a temperature-compensated conductivity.

An optional compressed air source 175, for example in the form of a compressor, may be provided at the machine side upstream of the blood filter 303.

A leakage sensor 167 is optionally provided. It may alternatively be provided at another place.

Further flow pumps in addition to or alternatively to, e.g., the one with the reference numeral 169 may also be provided.

A number of optional valves are each denoted with V in Fig. 1; by-pass valves with VB.

In some embodiments, the control device 150 determines the electrolyte balance and/or liquid balance based on the measurement values from the aforementioned optional sensors.

Filters F1 and F2 can be connected in series.

Even when using non-pure water, the filter F1 exemplarily serves herein to generate sufficiently pure dialysis liquid by the mixing device 163, which then flows through the blood filter 303, e.g., using the countercurrent principle.

The filter F2 exemplarily serves herein to generate sterile or sufficiently filtered substitute fluid from the sufficiently pure dialysis liquid leaving the first filter F1, by filtering e.g. pyrogenic substances. Said substitute fluid may then be safely added to the extracorporeally flowing blood of the patient and thus ultimately to the patient's body.

The treatment apparatus 100 is optionally shown in Fig. 1 as an apparatus for hemo(dia)filtration. However, hemodialysis apparatuses are also encompassed by the present invention, although possibly not specifically represented in a figure.

The arrows shown in Fig. 1 generally indicate the flow direction.

Furthermore, the control device 150 is, for the purpose of retrieving or transmitting data, for example adjustable settings and/or predicted data, in wired, or preferably wireless, signal communication (generally shown as dashed double arrows in Fig. 1) with the computer system 1 according to the present invention, which in the example of Fig. 1 may be physically separated from the hemodialysis treatment apparatus 100. The computer system 1 may comprise a calculation device 5 for executing the calculation steps, where applicable. Additionally, the computer system 1 may be connected in mutual signal communication with a central unit or cloud 7, from which e.g. input data may be retrieved. The central unit or cloud 7 may in some embodiments be or comprise the digital storage medium according to the present invention, or vice versa.

The control device 150 may be prepared to receive adjustable settings transmitted by signal transmission from the computer system 1. Alternatively or additionally, it may be programmed to control or control in a closed-loop manner the hemodialysis treatment apparatus 100 based on the adjustable settings transmitted via signal transmission.

The hemodialysis treatment apparatus 100 according to the present invention in the example of Fig. 1 further comprises a display 160, which is optionally provided and embodied for interacting with and/or informing the user. The display 160 may be designed as output device and/or as input device.

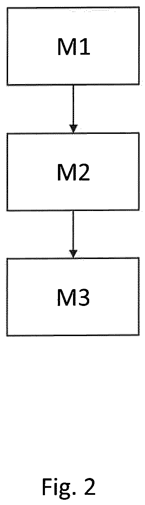

**Fig. 2** shows in an exemplary embodiment a flowchart of a computer-implemented method according to the present invention for predicting a patient's (P) fistula failure or for assessing the probability of the fistula to fail, e.g., within a preset time period or in the course of predetermined number of treatment sessions.

As a first method step **M1** the computer-implemented method comprises the inputting of a plurality of parameter values into a computer system 1 or a component thereof. Some of these values have been measured during one or more dialysis treatment sessions while treating the patient P using a hemodialysis treatment apparatus, e.g. the one of Fig. 1, or in connection with such dialysis treatment sessions, respectively.

Alternatively or additionally, some of these values are or have been calculated based on, or derived from, values that have been measured during one or more dialysis treatment sessions while treating the patient P using a hemodialysis treatment apparatus 100.

The values of the parameters which are input comprise or consist of, at least two or more of:
clearance,
sum pressure,
clearance/blood flow-ratio,
clearance/reference clearance-ratio.

The clearance is the capacity of the dialyzer 300 to clean the blood of *urea* or the urea removal rate and can be calculated based on urea measurements, preferably in as a in-vivo clearance measurement. For example, the Online Clearance Monitoring (OCM^{®}) system by Fresenius Medical Care, Germany, integrated, e.g., into their therapy system 5008, intradialytically measures the effective in-vivo urea clearance achieved during the treatment session.

The sum pressure is the sum of the, e.g. simultaneously, measured arterial pressure and venous pressure, e.g. the pressure measured by sensors PS1 or PS2 (Fig. 1) plus the pressure measured, preferably simultaneously, by sensor PS3.

The clearance/blood flow-ratio is the clearance divided by the blood flow or vice versa, and the clearance/reference clearance-ratio is the clearance divided by a reference clearance, or vice versa.

As a second method step **M2** the computer-implemented method encompasses predicting or assessing the estimated probability of an event predefined as a fistula failure within a predetermined time period. Thereby, the time period may start as of the time of the prediction or as of the time of executing/carrying out this method. Alternatively, an event may be predefined as a fistula failure within a predetermined number of, preferably subsequent, treatment sessions of the patient P.

In some embodiments of the computer-implemented method according to the present invention, the dynamic of the parameters during a treatment session of interest is not taken into account, but, for example, the median value of said parameter values measured during or calculated with respect to the treatment session.

For predicting the probability an artificial intelligence model, e.g., a machine learning model, may be used. Additionally or alternatively, a logistic regression model or a gradient boosting framework based on decision tree classifiers may be used.

The predicted probability may be considered the target variable or the dependent variable in statistics. A target variable is the outcome one aims to predict or explain using, e.g., a machine learning model. A target variable is the variable that is estimated or classified based on the input values.

Eventually, outputting the predicted probability or the result of the assessment is encompassed by the computer-implemented method as method step **M3.**

The probability or the result of the assessment may be output, for example to a printer, to a handheld device of any kind, e.g. a smartphone, a tablet etc. Additionally or alternatively, it may be output to the hemodialysis treatment apparatus 100.

Also, outputting suggestions regarding medical or surgical treatment based on the predicted probability or the result may be encompassed.

The present invention is not limited to the embodiments described herein. These are for illustrative purposes only.

### Reference Numerals

- 1: computer system
- 5: calculation device
- 7: central unit; cloud

- 100: hemodialysis treatment apparatus
- 101: blood pump
- 102: dialysate outlet line
- 104: dialysis liquid inlet line
- 105: substitute fluid line
- 107: predilution valve
- 107a: line associated with the predilution valve
- 109: postdilution valve
- 109a: line associated with the postdilution valve
- 111: substitute fluid pump
- 131: ultrafiltration pump

- 150: control device
- 153: outlet for effluent
- 155: water source
- 157: heat exchanger
- 159: first flow pump
- 160: display
- 161: device for balancing
- 162: heating device
- 163: mixing device
- 163a: conductivity sensor
- 163b: conductivity sensor
- 165a: temperature sensor
- 165b: temperature sensor
- 166: concentrate supply
- 167: leakage sensor
- 168: concentrate supply
- 169: second flow pump
- 171: pump; sodium pump
- 173: pump; bicarbonate pump
- 175: compressed air source
- 300: extracorporeal blood circuit
- 301: first line; arterial line section; blood withdrawal line; arterial patient line
- 302: arterial patient tubing clamp
- 303: blood filter or dialyzer
- 303a: dialysis liquid chamber
- 303b: blood chamber
- 303c: semi-permeable membrane
- 305: second line; venous line section; blood return line; venous patient line
- 306: venous patient tubing clamp
- 315: detector for detecting air bubbles and/or blood
- 317: single-needle chamber
- 318: de-aeration device
- 319: detector for detecting air bubbles and/or blood
- 325: addition site for anticoagulants, e.g. Heparin
- 329: venous air separation chamber; venous bubble trap (optional)

- A: container for A-concentrate; sodium
- B: container for B-concentrate; bicarbonate

- F1: filter
- F2: further filter level or stage

- M1 to M3: method steps
- P: patient

- PS1: arterial pressure sensor (optional)
- PS2: arterial pressure sensor (optional)
- PS3: pressure sensor (optional)
- PS4: pressure sensor (optional)
- PS5: pressure sensor

- V: valves
- V24: valve
- V25: valve
- VB: bypass valve

## Claims

1. A computer-implemented method for predicting a patient's (P) fistula failure or vascular, in particular venous, access failure comprising
- inputting, into a computer system (1), a plurality of parameter values,
some of which values having been measured during one or more dialysis treatment sessions while treating the patient (P) using a hemodialysis treatment apparatus (100); and/or
some of which values being or having been calculated based on or derived from values having been measured during one or more dialysis treatment sessions while treating the patient (P) using the hemodialysis treatment apparatus (100);
- predicting the probability of an event predefined as a fistula or vascular access failure within a predetermined time period or within a predetermined number of treatment sessions; and
- outputting the predicted probability.

2. The computer-implemented method according to claim 1, wherein the parameters for which values are input comprise or consist of, at least two or more of, the following ones:
- clearance (K)
- sum pressure
- clearance/blood flow-ratio
- clearance/reference clearance-ratio
with:
clearance being:
the capacity of the dialyzer to clean the blood of *urea* or the urea removal rate
sum pressure being:
the sum of the arterial pressure and the venous pressure
clearance/blood flow-ratio being:
the clearance divided by the blood flow or vice versa
clearance/reference clearance-ratio being:
the clearance divided by a reference clearance, or vice versa

3. The computer-implemented method according to any one of the preceding claims, wherein the dynamic of the parameters during a treatment session of interest is not taken into account, but, for example, the median value of said parameter values measured during or calculated with respect to the treatment session.

4. The computer-implemented method according to any one of the preceding claims, wherein the predicted probability is output to a printer, a display (160), a handheld device of any kind, e.g. a smartphone, a tablet etc., and/or to the hemodialysis treatment apparatus (100).

5. The computer-implemented method according to any one of the preceding claims, wherein for predicting the probability an artificial intelligence model, e.g., a machine learning model, is used.

6. The computer-implemented method according to the preceding claim, wherein a logistic regression model or a gradient boosting framework based on decision tree classifiers is used.

7. The computer-implemented method according to any one of the preceding claims, encompassing outputting suggestions regarding medical or surgical treatment based on the predicted probability or the result.

8. A computer system (1) programmed for carrying out/executing the computer-implemented method according to any one of claims 1 to 7.

9. The computer system (1) according to claim 8, configured for measuring, determining or retrieving a reference clearance and/or a reference, or being in signal communication with devices for measuring or storing reference clearance and/or reference values, or being prepared for being in signal communication with such devices.

10. A hemodialysis treatment apparatus (100) comprising
- components (101, 131) for carrying out a hemodialysis treatment;
- a control unit (150) for controlling the components (101, 131);
the apparatus (100) further comprising, or being in signal communication with,
- the computer system (1) according to claim 8 or 9.

11. The hemodialysis treatment apparatus (100) according claim 10, wherein the control unit (150) comprises or consists of the computer system (1), or vice versa.

12. The hemodialysis treatment apparatus (100) according to any one of claims 10 to 11, comprising
- measuring devices (PS1, PS2, PS3) for measuring the parameters the values of which are input; and/or
- a calculating device (5) for calculating a value of at least one of the parameter.

13. The hemodialysis treatment apparatus (100) according to any one of claims 10 to 12, wherein the control unit (150) is programmed to control the hemodialysis treatment apparatus (100) during treatment based on the output predicted probability.

14. The hemodialysis treatment apparatus (100) according to any one of claims 10 to 13, wherein the control unit (150) is programmed to output suggestions regarding medical or surgical treatment based on the predicted probability or the result.

15. A digital storage medium, in particular a floppy disk, memory card, CD or DVD, EPROM, FRAM or SSD, with electronically readable control signals, configured to interact with a conventional computer system such that it is reprogrammed into a computer system (1) according to claim 8 or 9 and/or with the controller of a conventional hemodialysis treatment apparatus such that it is turned or reprogrammed into a hemodialysis treatment apparatus (100) according to any one of claims 10 to 14.

16. A computer program product, as a signal wave or having a program code stored on a machine-readable carrier, for interacting with a conventional computer system such that it is reprogrammed into a computer system (1) according to claim 8 or 9 and/or a with the controller of a conventional hemodialysis treatment apparatus is reprogrammed into a hemodialysis treatment apparatus (100) according to any one of claims 10 to 14.

17. A computer program with a program code which, when executed on a computer, reprograms a conventional computer system into a computer system (1) according to claim 8 or 9 and/or reprograms the controller of a conventional hemodialysis treatment apparatus such that the conventional hemodialysis treatment apparatus is turned into a hemodialysis treatment apparatus (100) according to any one of claims 10 to 14.
